# EUROPEAN PATENT APPLICATION

(11) **EP 4 152 341 A1**
(43) Date of publication of application: **22.03.2023**
(21) Application number: 21197166.8
(22) Date of filing: 16.09.2021
(51) Int. Cl.: G16H 50/20

(54) **SYSTEMS AND METHODS FOR DETERMINING A CUTANEOUS LESION SCORE OF A COMPANION ANIMAL**

(71) Applicant: Mars, Incorporated, McLean, VA 22101 (US)
(72) Inventor: LANGON, Xavier, 30470 Aimargues (FR); MONTOYA, Mathieu, 30470 Aimargues (FR); GOURDON, Isabelle, 30470 Aimargues (FR)
(74) Representative: Nony

(57) **Abstract**

The present disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s),
- at least one lesion image of said companion animal body surface with at least one cutaneous lesion, and
- optionally at least one metadata relative to said companion animal, the method including at least the steps of:
a) operating said trained assessment module on said at least one lesion image and said at least one metadata of said companion animal, and
b) based on said assessment module, generating a score indicative of the cutaneous state of said companion animal.

## Description

### FIELD OF THE DISCLOSURE

The disclosure relates to companion animal health, and in particular to the study of cutaneous lesion(s) of a companion animal and to atopic dermatitis conditions. Various embodiments of this disclosure relate generally to machine-learning based techniques for monitoring companion animal health, and, more particularly, to systems and methods for identifying companion animal cutaneous lesions.

### BACKGROUND

In most companion animals, a healthy skin and coat indicates that an animal is generally in good health. As the skin and coat condition of a companion animal provides such an important visual impact, in particular to their owners, it is an ongoing aim in the art to assess the cutaneous state of an animal.

A companion animal having a skin condition is the single most common reason for a companion animal to be taken to a veterinary practice. It is suggested that 15% of the workload of such veterinary practices accounts for animals suffering from skin conditions. One of the main skin conditions that contributes to this statistic is atopic dermatitis, which is a common, genetically predisposed, inflammatory and pruritic skin disease. The diagnosis of atopic dermatitis may be complicated by variables such as variations in its clinical presentations, genetic factors, extent of the lesions, stage of the disease, secondary infections, as well as resemblance to other non-atopic related skin diseases.

It can be difficult to reliably classify cutaneous lesions and efficiently guide the owner of the companion animal. Dermatology is a complex medical territory as skin can reveal all the external and internal diseases. A clinical examination is usually needed to diagnose a skin condition, which is time-consuming process that requires a deep understanding of dermatology. Furthermore, usually, only symptomatic treatments are prescribed for the skin condition, which results in not treating the underlying skin condition.

In human medicine, dermoscopy can be a helpful diagnostic tool based on magnification of dermatological lesions. A software associated with images taken through this clinical examination may be able to recognize some primary lesions. Although, the use of dermoscopy on melanoma diagnostic and for the follow-up of the lesions seems to be well-known, dermoscopy begins only to be used in veterinary dermatology, as described in the articles Genovese, 2014, Scarampella, 2014, Zanna, 2015, Scarampella, 2015 and Zanna, 2017.

For example, a smartphone application, named "Atopy index", has been developed based on Canine Atopic Dermatitis Lesion Index (CADLI) and pruritus intensity. Such an application helps veterinarian practitioners to easily and quickly assess the Canine Atopic Dermatitis Extent and Severity Index (CADESI-4) before and after treatment. The owner of the companion animal identifies the lesions by coloring them on the application, which is based on 20 different areas of the body surface and 3 lesion types. The application allows obtaining the dermatological index in just a few minutes based on a validated severity scale, along with the severity index, and allows tracking the evolution of the total score and severity for each affected area at each visit. This application, however, is not designed for the diagnosis of atopic dermatitis, but only as an aid in its management for the owner.

Thus, there still remains a need for improving existing methods in the field of atopic dermatitis conditions, and for providing efficient, easy and reliable methods to assist an animal owner and/or a veterinarian practitioner in assessing the nature of cutaneous lesion(s) in companion animals.

Reference to any particular activity is provided in this disclosure only for convenience and not intended to limit the disclosure. A person of ordinary skill in the art would recognize that the concepts underlying the disclosed devices and methods may be utilized in any suitable activity. The disclosure may be understood with reference to the following description and the appended drawings, wherein like elements are referred to with the same reference numerals.

According to certain aspects of the disclosure, methods and systems are disclosed for determining a cutaneous lesion score of a companion animal. In an exemplary use case, a machine-learning model may determine the cutaneous lesion score of a companion animal. A computer system may be configured to first receive companion animal data corresponding to the companion animal. For example, the companion animal data may include at least one lesions global image. For example, the companion animal data may include at least one lesions global image and at least one metadata file. A trained assessment module may then analyze, or operate on, the companion animal data, where the trained assessment module generates a cutaneous lesion score based on the analyzing. The cutaneous lesion score may, for example, indicate at least one cutaneous state of the companion animal, where one of the at least one cutaneous state may include at least one atopic dermatitis condition. The computer system may then be configured to transmit the cutaneous lesion score to a device, which may belong to a veterinarian and/or the guardian of the companion animal.

The terminology used below may be interpreted in its broadest reasonable manner, even though it is being used in conjunction with a detailed description of certain specific examples of the present disclosure. Indeed, certain terms may even be emphasized below; however, any terminology intended to be interpreted in any restricted manner will be overtly and specifically defined as such in this detailed description section. Both the foregoing general description and the following detailed description are exemplary and explanatory only and are not restrictive of the features, as claimed

### SUMMARY

According to certain aspects of the disclosure, methods and systems are disclosed for determining a cutaneous lesion score of a companion animal.

According to a first aspect, the disclosure relates to a method for determining a cutaneous lesion score of a companion animal suspected to have an atopic dermatitis condition, using an assessment module trained beforehand to learn features indicative of an atopic dermatitis condition in a companion animal, based at least on a plurality of previously acquired images of companion animal body surfaces, some of these images having cutaneous lesion(s), the method comprising at least the steps of:
a) receiving companion animal data corresponding to said companion animal, wherein the companion animal data include at least one lesions global image of said companion animal body surface, and optionally at least one metadata relative to said companion animal and/or at least one lesion specific image of a cutaneous lesion of said companion animal,
b) operating said trained assessment module on the companion animal data, and
c) based on said assessment module, generating a cutaneous lesion score indicative of at least one cutaneous state of said companion animal.

According to another aspect, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s),
- at least one companion animal data including at least one lesions global image of said companion animal body surface, and
- optionally at least one metadata relative to said companion animal, and
- optionally at least one lesion specific image of a cutaneous lesion of said companion animal
the method including at least the steps of:
a) operating said trained assessment module on said at least one companion animal data, and
b) based on said assessment module, generating a cutaneous lesion score indicative of at least one cutaneous state of said companion animal.

According to an embodiment, the method according to the disclosure may not necessitate the use of at least one metadata relative to said companion animal.

According to another embodiment, the method according to the disclosure may necessitate the use of at least one metadata relative to said companion animal.

According to an embodiment, the method according to the disclosure may not necessitate the use of at least one lesion specific image of a cutaneous lesion of said companion animal.

According to another embodiment, the method according to the disclosure may necessitate the use of at least one lesion specific image of a cutaneous lesion of said companion animal. By crossing the data from the images and eventually the metadata, and by using said assessment module, trained beforehand, a score indicative of at least one cutaneous state of said companion animal can be obtained. The disclosure thus allows helping general veterinarian practitioners in their dermatologic diagnostic. The method may allow recognizing the location and semiology of the lesions, leads towards the dermatological diagnostic hypothesis that matches best with the clinical signs and gives the chance to companion animals to have more reliable and faster diagnostics and treatments.

According to another aspect, the disclosure relates to a method for training an assessment module to learn features indicative of an atopic dermatitis condition in a companion animal, using at least a plurality of previously acquired images of companion animal body surfaces, some of these images having cutaneous lesion(s), the method including:
- extracting at least one lesion feature from each previously acquired image,
- associating at least said image and said at least one lesion feature to an animal cutaneous state, and
- training the assessment module to learn said association.

According to another aspect, the present disclosure relates to a device for determining a cutaneous lesion score of a companion animal suspected to have an atopic dermatitis condition, the device comprising an assessment module trained beforehand to learn features indicative of an atopic dermatitis condition in a companion animal, based at least on a plurality of previously acquired images of companion animal body surfaces, some of these images having cutaneous lesion(s), said trained assessment module being configured to operate on the companion animal data wherein the companion animal data includes at least one lesions global image of said companion animal body surface, and optionally at least one metadata relative to said companion animal and/or at least one lesion specific image of a cutaneous lesion of said companion animal, and to generate a cutaneous lesion score indicative of at least one cutaneous state of said companion animal.

According to another aspect, the disclosure relates to a device for determining a cutaneous lesion score of a companion animal suspected to have an atopic dermatitis condition, using at least:
- at least one lesions global image of said companion animal body surface, and
- optionally, at least one metadata relative to said companion animal, and
- optionally, at least one lesion specific image of a cutaneous lesion of said companion animal
the device including an assessment module trained beforehand to learn features indicative of an atopic dermatitis condition in a companion animal, based at least on a plurality of previously acquired images of companion animal body surfaces, some of these images having cutaneous lesion(s),
said trained assessment module being configured to operate on said at least one lesion image and optionally said at least one metadata of said companion animal, and to generate a score indicative of at least one cutaneous state of said companion animal.

The disclosure thus provides an efficient and reliable digital tool to help veterinarian practitioners in their diagnostic.

According to another aspect, the disclosure relates to a computer program product for determining a cutaneous lesion score of a companion animal suspected to have an atopic dermatitis condition, using an assessment module trained beforehand to learn features indicative of an atopic dermatitis condition in a companion animal, based at least on a plurality of previously acquired images of companion animal body surfaces, some of these images having cutaneous lesion(s), the computer program product comprising a support and stored on this support instructions that can be read by a processor, these instructions being configured to:
a) receiving companion animal data corresponding to said companion animal, wherein the companion animal data includes at least one lesions global image of said companion animal body surface, and optionally at least one metadata relative to said companion animal and/or at least one lesion specific image of a cutaneous lesion of said companion animal,
b) operating said trained assessment module on the companion animal data, and
c) based on said assessment module, generating a cutaneous lesion score indicative of at least one cutaneous state of said companion animal.

According to another aspect, the disclosure relates to a computer program product for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s),
- at least one lesions global image of said companion animal body surface, and
- optionally at least one metadata relative to said companion animal, and
- optionally, at least one lesion specific image of a cutaneous lesion of said companion animal
the computer program product including a support and stored on this support instructions that can be read by a processor, these instructions being configured to:
a) operating said trained assessment module on said at least one lesions global image and optionally said at least one metadata of said companion animal and/or lesion specific image of a cutaneous lesion of said companion animal, and
b) based on said assessment module, generating a score indicative of at least one cutaneous state of said companion animal.

According to another aspect, the disclosure relates to a computer-readable medium having stored thereon the computer program product as defined above. According to another aspect, the disclosure relates to a computer-implemented method for determining a cutaneous lesion score of a companion animal suspected to have an atopic dermatitis condition, the method comprising: receiving at least one lesions global image of said companion animal body surface, and optionally at least one metadata relative to said companion animal and/or at least one lesion specific image of a cutaneous lesion of said companion animal and determining based on these data a cutaneous lesion score of said companion animal. In a particular embodiment, this method further comprises a step of transmitting the cutaneous lesion score of the companion animal to a mobile device.

According to another aspect, the disclosure relates to a computer-implemented method for determining a cutaneous lesion score of a companion animal suspected to have an atopic dermatitis condition, the method comprising: receiving at least one lesions global image of said companion animal body surface, and optionally at least one metadata relative to said companion animal and/or at least one lesion specific image of a cutaneous lesion of aid companion animal, determining based on these data a cutaneous lesion score of said companion animal and determining a recommendation to a pet owner based on the score. In an embodiment, said recommendation is a diet recommendation. In another particular embodiment, this method further comprises a step of transmitting the recommendation to a mobile device of the pet owner.

In another aspect, the present disclosure relates to a computer-implemented method for determining a cutaneous lesion score of a companion animal.

According to an exemplary embodiment, the disclosure relates to a computer-implemented method for determining a cutaneous lesion score of a companion animal, including:
- receiving companion animal data corresponding to the companion animal, wherein the companion animal data includes at least one lesions global image and optionally at least one metadata file and/or at least one lesion specific image of a cutaneous lesion;
- operating a trained assessment module on the companion animal data;
- based on the operating, generating the cutaneous lesion score, wherein the cutaneous lesion score indicates at least one cutaneous state of the companion animal; and
- transmitting the cutaneous lesion score to a device.

According to another exemplary embodiment, the disclosure relates to a computer-implemented method for determining a cutaneous lesion score of a companion animal, the method comprising:
- receiving companion animal data corresponding to the companion animal, wherein the companion animal data includes at least one lesion image and at least one metadata file;
- operating a trained assessment module on the companion animal data;
- based on the operating, generating the cutaneous lesion score, wherein the cutaneous lesion score indicates at least one cutaneous state of the companion animal; and
- transmitting the cutaneous lesion score to a device.

In an embodiment of the computer-implemented method according to the disclosure, the cutaneous lesion score assesses at least one other dermatitis condition, wherein the at least one other dermatitis condition may include at least one of: sarcoptic mange, demodicosis, bacterial overgrowth syndrome, Malassezia dermatitis, bacterial folliculitis, contact dermatitis, and/or mucocutaneous T cell lymphoma.

In an embodiment of the computer-implemented method according to the disclosure, the at least one cutaneous state may include at least one atopic dermatitis condition.

In an embodiment of the computer-implemented method according to the disclosure, the companion animal data further comprises at least one optical zoomed image of at least one lesion.

In an embodiment of the computer-implemented according to the disclosure, the companion animal data includes at least one global image, wherein the at least one global image includes at least one cutaneous lesion and/or an enlarged image of at least one area of interest, wherein the at least one area of interest includes the at least one cutaneous lesion.

In an embodiment of the disclosure, the computer-implemented method may further include:
- retrieving a previously stored cutaneous score;
- comparing the previously stored cutaneous score to the cutaneous lesion score; and
- based on the comparing, assessing a previous treatment curative effect.

In an embodiment of the computer-implemented method according to the disclosure, the operating of the trained assessment module on the companion animal data may further include:
- performing, by an object detection model, a recognition analysis on the at least one lesions image;
- based on the recognition analysis, forming, by the object detection model, at least one lesion feature vector;
- receiving, from a trained neural network, at least one image feature vector associated with at least one known animal cutaneous state;
- forming, by a metadata encoding module, at least one metadata vector based on the at least one metadata file; and
- determining, by an assessment neural network, the cutaneous lesion score based on the at least one lesion feature vector, the at least one image feature vector, and the at least one metadata vector.

In an embodiment of the computer-implemented according to the disclosure, the recognition analysis may include:
- identifying at least one lesion corresponding to the at least one lesion image; and
- associating at least one feature with the at least one lesion, wherein the at least one feature includes at least one of: a color, a surface, and/or an aspect.

In an embodiment of the computer-implemented method according to the disclosure, the assessment neural network includes a single-layer perceptron artificial neural network.

In an embodiment of the disclosure, the computer-implemented may further include:
- determining a recommendation based on the cutaneous lesion score; and
- transmitting the recommendation to the device.

In an embodiment of the computer-implemented method according to the disclosure, the recommendation may be at least one health recommendation, at least one nutritional recommendation, and/or at least one medical recommendation.

In an embodiment of the computer-implemented method according to the disclosure, the at least one health recommendation may include at least one of: at least one food product, at least one pet service, at least one supplement, at least one ointment, at least one drug, and/or at least one pet product.

In an embodiment of the computer-implemented method according to the disclosure, the at least one nutritional recommendation may include at least one instruction to feed the companion animal at least one of: at least one supplement and/or at least one food.

In an embodiment of the computer-implemented method according to the disclosure, the at least one medical recommendation may include at least one of: at least one ointment instruction and/or at least one drug instruction.

In one embodiment of the computer-implemented method according to the disclosure, the at least one metadata file may include at least one of physiological data and/or medical data.

In one embodiment of the computer-implemented method according to the disclosure, the physiological data may include at least one of: a breed, a species, an activity level, a medical history, a reproductive status, an age, a gender, a weight, a spayed or neutered status, a biological value from a biological sample, a body condition, a health status, a lifestyle, a habitat, coat information, and/or a risk factor.

In one embodiment of the computer-implemented method according to the disclosure, the medical data may include at least one of: an age of disease onset, an existence of previous episodes of hotspots, urticarial or angioedema, a presence of cortico-response pruritus, a medical history of chronic and/or recurrent dermatoses or otitis, scaling or dryness, at least one gastrointestinal sign, an indication of whether symptoms worsen after walking in grass, and/or an excess of hair loss.

In one embodiment of the computer-implemented method according to the disclosure, the trained assessment module is trained on a plurality of previously acquired cutaneous lesion images, the training including:
a) extracting at least one lesion feature from each of the plurality of previously acquired cutaneous lesion images;
b) associating the at least one lesion feature and the corresponding image of the plurality of previously acquired cutaneous lesion images with an animal cutaneous state; and
c) training the trained assessment module based on the associating.

In an embodiment of the computer-implemented method according to the disclosure, the trained assessment module may include at least one neural network.

In an embodiment of the computer-implemented method according to the disclosure, the training of the trained assessment module may further include:
- updating a weight of the at least one neural network according to the associating between the animal cutaneous state and the at least one lesion feature.

It is to be understood that both the foregoing summary and the following description are exemplary and explanatory only and are not restrictive of the disclosed embodiments, as claimed.

### BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.
**Figure** 1 depicts a flowchart of an exemplary method of using a trained machine-learning model to determine a cutaneous lesion score for a companion animal suspected to have an atopic dermatitis condition according to one or more embodiments.
**Figure 2** depicts an example of a machine-learning model for determining a cutaneous lesion score, according to one or more embodiments.
**Figure 3** depicts an example of steps and elements implemented in an exemplary method, according to one or more embodiments.
**Figure 4** depicts an example of some of the most commonly affected areas in a dog with atopic dermatitis condition, according to one or more embodiments.
**Figure 5** depicts an example of some of the most commonly affected areas in a cat with an atopic dermatitis condition., according to one or more embodiments.

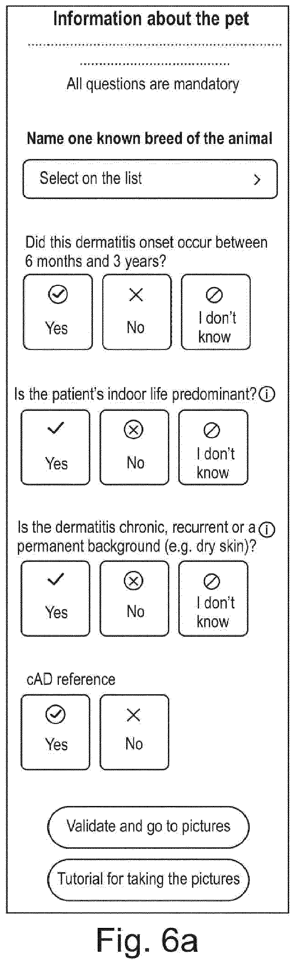

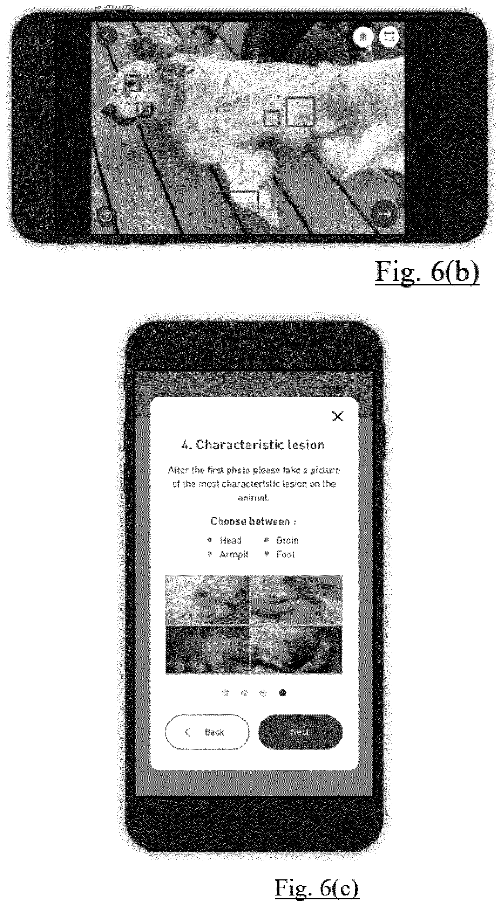

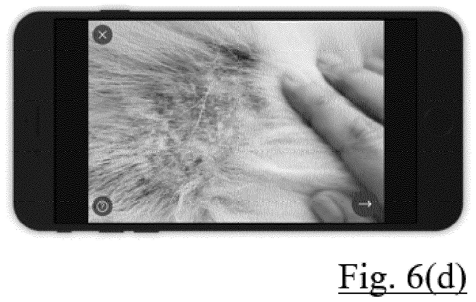

**Figure 6** shows an example of use of the method according to one or more embodiments, with Figure 6a depicting an example of a user interface allowing for input data, such as companion animal metadata, according to one or more embodiments; Figure 6b depicting an example of a user interface displaying a global image of the body surface of the companion animal, according to one or more embodiments; Figure 6c depicting an example of a user interface displaying the most characteristic lesions of a companion animal, according to one or more embodiments; and Figure 6d depicting an example of a user interface displaying the enlarged lesion images of the companion animal body surface.

### DETAILED DESCRIPTION

### Definitions

In the detailed description herein, references to "embodiment," "an embodiment," "one non-limiting embodiment," "in various embodiments," etc., indicate that the embodiment(s) described can include a particular feature, structure, or characteristic, but every embodiment might not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

In general, terminology can be understood at least in part from usage in context. For example, terms, such as "and", "or", or "and/or," as used herein can include a variety of meanings that man depend at least in part upon the context in which such terms are used. Typically, "or" if used to associate a list, such as A, B or C, is intended to mean A, B, and C, here used in the inclusive sense, as well as A, B or C, here used in the exclusive sense. In addition, the term "one or more" as used herein, depending at least in part upon context, can be used to describe any feature, structure, or characteristic in a singular sense or can be used to describe combinations of features, structures or characteristics in a plural sense.

Similarly, terms, such as "a," "an," or "the," again, can be understood to convey a singular usage or to convey a plural usage, depending at least in part upon context. In addition, the term "based on" can be understood as not necessarily intended to convey an exclusive set of factors and can, instead, allow for existence of additional factors not necessarily expressly described, again, depending at least in part on context.

The terms "having," "including," "containing" and "comprising" or any other variation thereof, are interchangeable, and one of skill in the art will recognize that these terms are open ended terms. They are intended to cover a non-exclusive inclusion, such that a process, method, article, or apparatus that comprises a list of elements does not include only those elements but can include other elements not expressly listed or inherent to such process, method, article, or apparatus.

As used herein, the term "about" or "approximately" means within an acceptable error range for the particular value as determined by one of ordinary skill in the art, which will depend in part on how the value is measured or determined, i.e., the limitations of the measurement system.

As used herein, the term "companion animal" or "pet" may include, for example, without limitation, a companion mammal. For example, companion mammals may encompass canines, felines, dogs, cats, rabbits, hamsters, guinea pigs, rats and/or mice. Preferred companion animals herein, without limitation, are canine or feline, especially as dogs and cats, in particular dogs.

As used herein, the term "mammal" or "mammals" may include, for example, without limitation, a human or an animal. In particular, the term "animal" or "animals" may include, for example, without limitation, a ruminant, a poultry, a swine, a mammal, a horse, a mouse, a rat, a rabbit, a guinea pig, a hamster, a cow, a cat, or a dog, preferably a companion animal, i.e., a cat and/or a dog.

As used herein, the term "adult" may include, for example, without limitation, an animal that has passed puberty or has reached its biological maturation point, or both.

As used herein, the term "canine" may include animals, including companion animals selected from recognized dog breeds (some of which are further subdivided), which may include Afghan hound, airedale, akita, Alaskan malamute, basset hound, beagle, Belgian shepherd, bloodhound, border collie, border terrier, borzoi, boxer, bulldog, bull terrier, cairn terrier, chihuahua, chow, cocker spaniel, collie, corgi, dachshund, dalmatian, doberman, English setter, fox terrier, German shepherd, golden retriever, great Dane, greyhound, griffon bruxellois, Irish setter, Irish wolfhound, King Charles spaniel, Labrador retriever, lhasa apso, mastiff, newfoundland, old English sheepdog, papillion, Pekingese, pointer, pomeranian, poodle, pug, rottweiler, St. Bernard, saluki, samoyed, schnauzer, Scottish terrier, Shetland sheepdog, shih tzu, Siberian husky, Skye terrier, springer spaniel, West Highland terrier, whip companion, Yorkshire terrier, etc.

As used herein, the term "feline" may include animals, including companion animals, selected from, without limitation, cheetah, puma, jaguar, leopard, lion, lynx, liger, tiger, panther, bobcat, ocelot, smilodon, caracal, serval and cats. As used herein, cats encompass wild cats and domestic cats, and most preferably domestic cats.

As used herein, a "subpopulation" may include, for example, without limitation, a set of one to several animals of one species, but less than an entire species. For example, "subpopulation" may be definable in terms of genotype and/or one or more attributes of physiological condition that, in a subpopulation of more than one member, are common to members of the subpopulation. In certain embodiments, the subpopulation may be defined at least in part by specific breed. Additionally, for example, in the case of animals of mixed breed, a subpopulation can be defined at least in part by breed heritage, which may be established through knowledge of the parental breeds, phenotypic characteristics, genotypic assessment, or by genetic markers such as SNPs. In certain embodiments, the subpopulation may be defined at least in part by physiological condition.

As used herein, the term "companion animal data" or "animal data" may include for example, without limitation, at least one global lesions image. In some embodiment, the companion animal data may further include at least one metadata. In some embodiment, the companion animal data may further include at least one specific lesion image. In another embodiment, the companion animal data may include at least one metadata and at least one specific lesion image.

As used herein, the term "lesions global image" may include for example, without limitation, at least an image of the companion animal body surface. In a preferred embodiment, the lesions global image may include at least one cutaneous lesion. The lesions global image may include at least one of the following companion animal body surface areas: a head, an ear, a perioral and/or periocular area, a leg, a front foot, an interdigital area, a flexor surface of the tarsal joint and/or an extensor surface of the carpal joint, a trunk, a groin, a ventral and/or perineal area, and/or an armpit. The lesions global image may include at least one image of an underside body surface of the companion animal.

As used herein, the term "lesion specific image" may include for example, without limitation, at least one cutaneous lesion and/or an enlarged image of at least one area of interest. The at least one area of interest may include at least one cutaneous lesion. In addition, the at least one lesion specific image may also include at least one optical zoomed image of at least one cutaneous lesion. The optical zoomed image may provide additional efficiency and reliability of the process.

When used without the distinction global or specific, the term "lesion image" or "lesions image" or "image(s)" is applicable to both the lesions global image and the lesion specific image.

As used herein, the term "metadata" or "metadata file" may include, for example, without limitation, any one or combination of attributes of an animal including at least its breed, species, activity level, medical history, reproductive status, age, gender, weight, spayed or neutered status, a biological value from a biological sample, body condition, health status, lifestyle, habitat, coat information, or risk factor, and/or medical data, such as the age of disease onset, the existence of previous episodes of hotspots, urticarial or angioedema, the presence of cortico-response pruritus, the excess of hair loss, scaling or dryness, gastrointestinal signs, an indication on whether or not symptoms worsen after walking in grass, and/or medical history of chronic and/or recurrent dermatoses or otitis.

Additionally, for example, the metadata may be composed of answers to questions pertaining to, but not limited to, the list of attributes above.

As used herein, the term "assessment module" may include, for example, without limitation, a module associated with a learning model and learning algorithms that analyze data, used in particular for classification and regression analysis.

As used herein, the term "expert" may include, for example, without limitation, a person able to identify cutaneous lesions on an animal body surface, to label features associated to such lesions, to discriminate such lesions and/or to associate them to an atopic dermatitis condition, other skin conditions, or other skin marks. Such an expert may be, for example, a veterinarian practitioner.

As used herein, the term "biological sample" or "biological material" may include, for example, without limitation, at least one of stool, urine, hair, blood, saliva, and tissue.

For example, the term "biological sample" or "biological material" may refer to a sample of tissue or fluid isolated from a subject, including but not limited to, for example, blood, plasma, serum, fecal matter, urine, bone marrow, bile, spinal fluid, lymph tissue and lymph fluid, samples of the skin, external secretions of the skin, respiratory, intestinal, and genitourinary tracts, tears, saliva, milk, blood cells, organs and/or biopsies. The term "biological sample" or "biological material" may also refer to samples of in vitro cell culture constituents including but not limited to conditioned media resulting from the growth of cells and tissues in culture medium, e.g., recombinant cells, and cell components. The term "biological sample" or "biological material" can also refer to, for example, without limitation, a polypeptide or a polynucleotide, or fragmented portions of organisms or cells obtained from sampling the environment, such as airborne pathogens.

As used herein, the term "food product" or "food composition" or "diet" or "foodstuff' may refer to, for example, without limitation foodstuff, diet, food supplement, liquid and/or a material that may contain proteins, carbohydrates and/or crude fats. For example, the term may also refer to supplementary substances or additives, for example, minerals, vitamins, and condiments (See Merriam- Webster's Collegiate Dictionary, 10th Edition, 1993, the entirety of which is hereby incorporated herein by reference). Such food compositions or products may be nutritionally complete or not.

As used herein, a "companion food" or "animal food" may include, for example, without limitation, a product produced by a companion food manufacturer, whether processed, partially processed or unprocessed, and/or intended to be ingested by companion animals after placing on the market; according to European Union (EU) Regulation n°767/2009.

As used herein, a "training data set" or "training data" can include one or more images or videos and associated data to train a machine learning model. Each training data set can comprise a training image of one or more products, data, and a corresponding output associated with the image. A training data set can include one or more images or videos of a plurality of previously acquired cutaneous lesion images. A training data set can be collected via one or more client devices (e.g., crowd-sourced) or collected from other sources (e.g., a database). In certain non-limiting embodiments, the training data set for a wellness assessment of a pet can include data from both a treatment group and a control group.

Certain non-limiting embodiments are described below with reference to block diagrams and operational illustrations of methods, processes, devices, and apparatus. It is understood that each block of the block diagrams or operational illustrations, and combinations of blocks in the block diagrams or operational illustrations, can be implemented by means of analog or digital hardware and computer program instructions. These computer program instructions can be provided to a processor of a general-purpose computer to alter its function as detailed herein, a special purpose computer, ASIC, or other programmable data processing apparatus, such that the instructions, which execute via the processor of the computer or other programmable data processing apparatus, implement the functions/acts specified in the block diagrams or operational block or blocks. In some alternate implementations, the functions/acts noted in the blocks can occur out of the order noted in the operational illustrations. For example, two blocks shown in succession can in fact be executed substantially concurrently or the blocks can sometimes be executed in the reverse order, depending upon the functionality/acts involved.

In certain non-limiting embodiments, the term "server" should be understood to refer to a service point which provides processing, database, and communication facilities. By way of example, and not limitation, the term "server" can refer to a single, physical processor with associated communications and data storage and database facilities, or it can refer to a networked or clustered complex of processors, such as an elastic computer cluster, and associated network and storage devices, as well as operating software and one or more database systems and application software that support the services provided by the server. The server, for example, can be a cloud-based server, a cloud-computing platform, or a virtual machine. Servers can vary widely in configuration or capabilities, but generally a server can include one or more central processing units and memory. A server can also include one or more mass storage devices, one or more power supplies, one or more wired or wireless network interfaces, one or more input/output interfaces, or one or more operating systems, such as Windows Server, Mac OS X, Unix, Linux, FreeBSD, or the like.

For some non-limiting embodiments, a "network" should be understood to refer to a network that may couple devices so that communications can be exchanged, such as between a server and a client device or other types of devices, including between wireless devices coupled via a wireless network, for example. A network can also include mass storage, such as network attached storage (NAS), a storage area network (SAN), or other forms of computer or machine-readable media, for example. A network can include the Internet, one or more local area networks (LANs), one or more wide area networks (WANs), wire-line type connections, wireless type connections, cellular or any combination thereof. Likewise, sub-networks, which can employ differing architectures or can be compliant or compatible with differing protocols, can interoperate within a larger network. Various types of devices can, for example, be made available to provide an interoperable capability for differing architectures or protocols. As one illustrative example, a router can provide a link between otherwise separate and independent LANs.

A communication link or channel can include, for example, analog telephone lines, such as a twisted wire pair, a coaxial cable, full or fractional digital lines including T1, T2, T3, or T4 type lines, Integrated Services Digital Networks (ISDNs), Digital Subscriber Lines (DSLs), wireless links including satellite links, or other communication links or channels, such as can be known to those skilled in the art. Furthermore, a computing device or other related electronic devices can be remotely coupled to a network, such as via a wired or wireless line or link, for example.

In certain non-limiting embodiments, a "wireless network" should be understood to couple client devices with a network. A wireless network can employ standalone ad-hoc networks, mesh networks, wireless land area network (WLAN), cellular networks, or the like. A wireless network may be configured to include a system of terminals, gateways, routers, or the like coupled by wireless radio links, or the like, which can move freely, randomly, or organize themselves arbitrarily, such that network topology can change, at times even rapidly. A wireless network can further employ a plurality of network access technologies, including Wi-Fi, Long Term Evolution (LTE), WLAN, Wireless Router (WR) mesh, or 2nd, 3rd, 4th, 5th generation (2G, 3G, 4G, or 5G) cellular technology, or the like. Network access technologies can allow wide area coverage for devices, such as client devices with varying degrees of mobility, for example. For example, a network may be configured to provide radio frequency (RF) or wireless type communication via one or more network access technologies, such as Global System for Mobile communication (GSM), Universal Mobile Telecommunications System (UMTS), General Packet Radio Services (GPRS), Enhanced Data GSM Environment (EDGE), 3GPP LTE, LTE Advanced, Wideband Code Division Multiple Access (WCDMA), Bluetooth, 802. llb/g/n, or the like. A wireless network can include virtually any type of wireless communication mechanism by which signals can be communicated between devices, such as a client device or a computing device, between or within a network, or the like. A computing device can send or receive signals, such as via a wired or wireless network, or can process or store signals, such as in memory as physical memory states. For example, a computing device can operate as a server and can include, as examples, dedicated rack-mounted servers, desktop computers, laptop computers, set top boxes, integrated devices combining various features, such as two or more features of the foregoing devices, or the like. Servers can vary widely in configuration or capabilities, but generally a server can include one or more central processing units and memory. A server can also include one or more mass storage devices, one or more power supplies, one or more wired or wireless network interfaces, one or more input/output interfaces, or one or more operating systems.

As used herein, a "machine-learning model" generally encompasses instructions, data, and/or a model configured to receive input, and apply one or more of a weight, bias, classification, or analysis on the input to generate an output. The output may include, for example, a classification of the input, an analysis based on the input, a design, process, prediction, or recommendation associated with the input, or any other suitable type of output. A machine-learning model is generally trained using training data, e.g., experiential data and/or samples of input data, which are fed into the model in order to establish, tune, or modify one or more aspects of the model, e.g., the weights, biases, criteria for forming classifications or clusters, or the like. Aspects of a machine-learning model may operate on an input linearly, in parallel, via a network (e.g., a neural network), or via any suitable configuration. The execution of the machine-learning model may include deployment of one or more machine learning techniques, such as linear regression, logistical regression, random forest, gradient boosted machine (GBM), deep learning, and/or a deep neural network. Supervised and/or unsupervised training may be employed. For example, supervised learning may include providing training data and labels corresponding to the training data, e.g., as ground truth. Unsupervised approaches may include clustering, classification or the like. K-means clustering, or K-Nearest Neighbors may also be used, which may be supervised or unsupervised. Combinations of K-Nearest Neighbors and an unsupervised cluster technique may also be used. Any suitable type of training may be used, e.g., stochastic, gradient boosted, random seeded, recursive, epoch or batch-based, etc. In an exemplary use case, a machine-learning model may determine a cutaneous lesion score of a companion animal. The computer system may be configured to first receive companion animal data corresponding to the companion animal. For example, the companion animal data may include at least one lesion image and at least one metadata file. A trained assessment module may then analyze, or operate on, the companion animal data, where the trained assessment module generates a cutaneous lesion score based on the analyzing. The cutaneous lesion score may, for example, indicate at least one cutaneous state of the companion animal, where one of the at least one cutaneous states may include at least one atopic dermatitis condition. The computer system may then be configured to transmit the cutaneous lesion score to a device, which may belong to a veterinarian and/or the guardian of the companion animal. In another exemplary use case, a machine-learning model, such as a trained assessment module, may be trained to generate a cutaneous lesion score. The training may include utilizing training data, such as a plurality of previously acquired cutaneous lesion images. For example, the trained assessment module may extract at least one lesion feature from each of the plurality of previously acquired cutaneous lesion images. The trained assessment module may then associate the at least one lesion feature and the corresponding cutaneous lesion image with a companion animal cutaneous state. This process may continue until an association may be made for each of the previously acquired cutaneous lesion images. The trained assessment module may then be further trained based on such associations.

While the examples above involve cutaneous lesions and atopic dermatitis conditions, it should be understood that techniques according to this disclosure may be adapted to any suitable type of companion animal health analysis. It should also be understood that the examples above are illustrative only. The techniques and technologies of this disclosure may be adapted to any suitable activity.

### Method for determining a cutaneous lesion score of a companion animal

According to an aspect, the disclosure relates to a method for determining a cutaneous lesion score of a companion animal suspected to have an atopic dermatitis condition, using an assessment module trained beforehand to learn features indicative of an atopic dermatitis condition in a companion animal, based at least on a plurality of previously acquired images of companion animal body surfaces, some of these images having cutaneous lesion(s), the method comprising at least the steps of:
a) receiving companion animal data corresponding to said companion animal, wherein the companion animal data include at least one lesions global image of said companion animal body surface, and optionally at least one metadata relative to said companion animal and/or at least one lesion specific image of a cutaneous lesion of said companion animal,
b) operating said trained assessment module on the companion animal data, and
c) based on said assessment module, generating a cutaneous lesion score indicative of at least one cutaneous state of said companion animal.

According to an aspect, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s), and
- at least one lesions global image of said companion animal body surface, and
- optionally at least one metadata relative to said companion animal, and
- optionally at least one lesion specific image of a cutaneous lesion of said companion animal
the method including at least the steps of:
a) operating said trained assessment module on said at least one lesions global image and optionally said at least one metadata and/or one lesion specific image of said companion animal, and
b) based on said assessment module, generating a score indicative of at least one cutaneous state of said companion animal.

In one embodiment, said companion animal is a canine or a feline.

In one embodiment, said companion animal is a dog or a cat.

In one embodiment, said companion animal is a dog.

### Companion animal data

In one embodiment, the method according to the disclosure may use at least one companion animal data, said companion animal data comprising at least a lesions global image of said companion animal body surface. Areas of said companion animal body surface may be chosen among the head, especially the ear, perioral and/or periocular areas, the legs, especially the front feet and/or the interdigital areas, the flexor surface of the tarsal joint and/or the extensor surface of the carpal joint, and the trunc, especially the groin, armpit, ventral and/or perineal areas.

In one embodiment, said at least one lesions global image may be an image of the underside body surface of said companion animal.

An additional lesion specific image may be used, especially an optical zoomed image of said cutaneous lesion. This allows increasing the efficiency and reliability of the method.

In one embodiment of the disclosure, said images may be taken by the general veterinarian practitioner during the consultation. In another embodiment, said images may be taken by the pet owner or the pet parent or any other person. Said images may be taken by any photographic device, such as a camera or, in a particular embodiment, the camera of a smartphone device.

In a variant, and more particularly regarding to the lesion specific image, a microscopic device adapted to a smartphone may be used, such as a microscope added on the lens of a smartphone camera. Such devices bring a strong reliability and a good magnification.

According to another embodiment, the method according to the disclosure may necessitate the use of at least one metadata relative to said companion animal.

According to said embodiment, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s), and
- at least one lesions global image of said companion animal body surface, and
- at least one metadata relative to said companion animal,
the method including at least the steps of:
a) operating said trained assessment module on said at least one lesions global image and said at least one metadata of said companion animal, and
b) based on said assessment module, generating a score indicative of at least one cutaneous state of said companion animal.

Said at least one metadata relative to said companion animal may include, for example, without limitation:
- physiological data, referring to any one or combination of attributes of the companion animal such as breed, species, sex, size, activity level, medical history, reproductive status, age, gender, weight, spayed or neutered status, a biological value from a biological sample, body condition, health status, lifestyle, habitat, coat information, or risk factor, and/or
- medical data, referring to any one or combination of diagnosed pathology(ies) or medical condition(s) as determined by a veterinary assessment such as the age of disease onset, the existence of previous episodes of hotspots, urticaria or angioedema, the presence of cortico-response pruritus, the excess of hair loss, scaling or dryness, gastrointestinal signs, an indication on whether or not symptoms worsen after walking in grass, or medical history of chronic and/or recurrent dermatoses or otitis.

The metadata thus advantageously can come from disease history and animal characteristics. Their use in the method according to the disclosure may allow increasing the specificity and sensitivity of the method. The metadata may be composed of answers to questions asked to the owner and/or to the veterinarian practitioner.

The method according to the disclosure may use a plurality of metadata relative to said companion animal, and especially including the step of providing a pathological profile of said companion animal based on said plurality of metadata.

According to another embodiment, the method according to the disclosure may necessitate the use of at least one lesion specific image of a cutaneous lesion of said companion animal.

According to said embodiment, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s), and
- at least one lesions global image of said companion animal body surface, and
- at least one lesion specific image of a cutaneous lesion of said companion animal,
the method including at least the steps of:
a) operating said trained assessment module on said at least one lesion global image and said at least one lesion specific image, and
b) based on said assessment module, generating a score indicative of at least one cutaneous state of said companion animal.

According to another embodiment, the method according to the disclosure may necessitate the use of at least both one metadata and one lesion specific image of a cutaneous lesion of said companion animal.

According to said embodiment, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s), and
- at least one lesions global image of said companion animal body surface, and
- at least one metadata relative to said companion animal, and
- at least one lesion specific image of a cutaneous lesion of said companion animal,
the method including at least the steps of:
a) operating said trained assessment module on said at least one lesions global image and said at least one metadata and at least one lesion specific image, and
b) based on said assessment module, generating a score indicative of at least one cutaneous state of said companion animal.

According to another particular embodiment, the method according to the disclosure may not necessitate the use of at least one metadata relative to said companion animal nor one lesion specific image of a cutaneous lesion of said companion animal.

According to said embodiment, the disclosure relates to a method for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s), and
- at least one lesions global image of said companion animal body surface, and
the method including at least the steps of:
a) operating said trained assessment module on said at least one lesions global image of said companion animal, and
b) based on said assessment module, generating a score indicative of the cutaneous state of said companion animal.

### Assessment module

In a particular embodiment, said assessment module can use a predictive model.

In a particular embodiment, said predictive model can include an object detection model, a neural network, especially a convolutional neural network, and a metadata encoding module.

In one particular embodiment, said object detection model can be real-time, can be based on YOLOv3 with the DarkNet architecture, as described in https://arxiv.org/abs/1506.02640 and https://pjreddie.com/darknet/yolo/, and can have been finetuned and trained to reference cutaneous lesions on the images.

Said object detection model can advantageously perform a recognition analysis on said at least one lesion image to reference said at least one cutaneous lesion, one or more lesion features being especially referenced, such as spatial features, as the origin coordinates, the height, and the width.

Said lesion features can be labelled by an expert.

In a variant embodiment, the lesion features are recognized by using an object detection algorithm.

In such an embodiment, a clinical criterion can be used, as a pattern criterion relative to areas most commonly affected or unaffected by atopic dermatitis, such as affected head, in particular perioral or periocular lesions or affected concave face of ear pinnae, affected limbs, in particular affected front feet and/or interdigital area, bilaterally affected limbs, pododermatitis, or dermatitis of the flexor surface of the tarsal joint and/or the extensor surface of the carpal joint, affected trunc, in particular inguinal or axillar lesions, underside of abdomen and perineal lesions, and/or non-affected ear margins or non-affected dorso-lumbar area. In a variant or in combination, said clinical criterion can be a lesion criterion, such as a value corresponding to erythema/erythroderma, papules/plaques, wheal, alopecia, lichenification, pigmentary abnormality, scale, crust, seborrhea, or ulcer/erosion/excoriation.

In an embodiment, each image is transformed in an image feature vector, especially by using a convolution of the pixels. In a particular embodiment where the images have a size of 256^{∗}256 pixels with 3 color layers, said image is transformed into an image feature vector of size 4096^{∗}1. Said object detection model advantageously only uses the pixels of the color image and the referenced lesion features.

This step allows identifying the lesion(s) on the tested image and associating some features to it. The color, surface, and aspect of the lesion are advantageously used in the assessment thanks to said image vector comprising the color pixels of the image.

A lesion feature vector may be formed, corresponding to the vector used to detect said lesion features, in particular spatial features as the origin coordinates, the width and the height of the identified lesion(s).

Said convolutional neural network may be based on the opensource model InceptionV3, which is the third edition of Google's Inception Convolutional Neural Network trained on Image Net dataset. In a particular embodiment, said convolutional neural network can be trained beforehand to recognize cutaneous states of companion animals based at least one lesion feature extracted from said plurality of previously acquired images of companion animal body surfaces.

Image feature vectors associated to known animal cutaneous states are advantageously formed.

Said metadata encoding module can be advantageously configured to convert said metadata, especially the answers to questions as defined above, into a categorical vector. The list of questions having a set of predefined answers, said metadata encoding module advantageously uses a binarization function to attribute a 1 or a 0 to each possible answer leading to a metadata vector.

In a particular embodiment, said assessment module may further include an assessment neural network, especially a single-layer perceptron artificial neural network.

Said assessment neural network advantageously can use said lesion feature vector of object detection model, said image feature vector(s) of said convolutional neural network and said metadata vector to generate the score indicative of the cutaneous state of said companion animal.

Said vectors can be each associated to a companion animal and to the known cutaneous state of said animal, as presented in the table below.

**Table 1**

| | | | | | |
|---|---|---|---|---|---|
| Animal 1 | Lesion feature vector | Image feature vector | Metadata vector | | Cutaneous State |
| Animal 2 | Lesion feature vector | Image feature vector | Metadata vector | | Cutaneous State |
| Animal 3 | Lesion feature vector | Image feature vector | Metadata vector | | Cutaneous State |
| Animal 4 | Lesion feature vector | Image feature vector | Metadata vector | | Cutaneous State |
| | | | | | |
| Animal N | Lesion feature vector | Image feature vector | Metadata vector | | Cutaneous State |

The number of neurons of single layer of said assessment neural network can be optimized and the connection weights of said assessment neural network can be refined for the training in order to minimize the errors.

Said vectors may be combined into one global matrix of features, used by said assessment neural network to generate the score.

### Cutaneous lesion score

In an embodiment, said generated score depends at least on an assessed correlation between said previously acquired images and said lesion image.

Said score may be a probability, or a percentage, showing the level of confidence for the lesion(s) to be indicative of an atopic dermatitis condition, for example low, medium or high.

In an embodiment, said score may be in the form of a numerical value, for example a value of 0 (no dermatitis condition) or 1 (dermatitis condition).

In another embodiment, said score may be in the form of a numerical value, for example a value between 0 and 10. In another embodiment, said score may be in the form of a letter, especially showing that the cutaneous state of said companion animal is thought to belong to a type of cutaneous states, for example group A, group B, group C, and so on.

In yet another embodiment, said score may be a class indicative of the cutaneous state of said companion animal, or a class indicative of a dermatitis condition in said companion animal, atopic or otherwise, or a combination thereof, for example a class and a probability that the animal belongs to said class.

The method according to the disclosure may further include the step of providing to a user interface said score relative to said companion animal and generated at step b).

Said score(s) may be transmitted to a user by any suitable mean, for example by being displayed on a screen of an electronic device, printed, or by vocal synthesis.

Said score(s) may be used as entry value in another program, and/or maybe combined to other information, for example clinical and/or biological data.

The method according to the disclosure may suggest some complementary analysis to further refine the score. A suggestion of the best diet associated with each score may also be provided.

Said score relative to said companion animal and generated at step b) may further assess the severity evolution of said atopic dermatitis condition.

In an embodiment, if said score relative to said companion animal and generated at step b) assessing that said at least one cutaneous lesion is not indicative of an atopic dermatitis condition in said companion animal, said score assesses other dermatitis conditions such as sarcoptic mange, demodicosis, bacterial overgrowth syndrome, *Malassezia* dermatitis, bacterial folliculitis, contact dermatitis, or mucocutaneous T cell lymphoma. In such an embodiment, lesion features and/or clinical criterions different than the ones used for atopic dermatitis condition can be used, in order to best describe such other dermatitis conditions.

In an embodiment, a previous score relative to the same companion animal being stored, the method further includes the step of comparing said previous score to the score generated at step b) in order to assess the curative effect of an adopted treatment method.

According to another embodiment, the disclosure relates to a method for assessing the nature of cutaneous lesion(s) of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s),
- at least one lesions global image of said companion animal body surface, and
- optionally, at least one metadata relative to said companion animal, and
- optionally, at least one lesion specific image of a cutaneous lesion of said companion animal,
the method including at least the steps of:
a) operating said trained assessment module on said at least one lesions global image and, optionally, said at least one metadata and/or lesion specific image of said companion animal, and
b) based on said assessment module, generating a score assessing at least whether or not said at least one cutaneous lesion is indicative of an atopic dermatitis condition in said companion animal.

### Method for training an assessment module to learn features indicative of an atopic dermatitis condition in a companion animal

According to another aspect, the disclosure relates to a method for training an assessment module to learn features indicative of an atopic dermatitis condition in a companion animal, using at least a plurality of previously acquired images of companion animal body surfaces, some of these images having cutaneous lesion(s), the method including:
- extracting at least one lesion feature from each previously acquired image,
- associating at least said image and said at least one lesion feature to an animal cutaneous state, and
- training the assessment module to learn said association.

The lesion features can be outlined and labeled by an expert. A bookmark may be added on the lesions.

In a particular embodiment, for the learning, not all images are of companion animals with cutaneous lesions, but some images can be of animals which are not affected with cutaneous lesions, and some can be of animals with lesions due to conditions other than atopic dermatosis. The global learning dataset preferably can contain atopic animals, animals affected with other dermatosis and healthy animals.

In one embodiment, said assessment module may include at least one neural network, the method including the step of updating weights of the neural network according to said association between said at least one lesion feature and said animal cutaneous state.

The assessment module may be configured to assign weights to each of the inputs during the learning phase, the weights optimization being especially carried out by a solver of the "stochastic gradient descent" type.

In one embodiment, said association between said at least one lesion feature and said animal cutaneous state may be used to modify atopic dermatitis features of reference for a future iteration of the method, in order to improve the accuracy and reliability of the method according to the disclosure.

The features described above for the method for determining a cutaneous lesion score can apply to the method for training an assessment module, and vice and versa.

### Devices & Computer-implemented methods & mediums

The provided methods can be computer-implemented methods.

Hence, in one embodiment, the provided methods for determining a cutaneous lesion score of a companion animal suspected to have an atopic dermatitis condition and/or for training an assessment module to learn features indicative of an atopic dermatitis condition in a companion animal can be achieved either offline, i.e., not controlled by a device such as a computer-aided system; or alternatively online, i.e. controlled by a computer-aided system, such as one including a device suitable for determining a cutaneous lesion score of a companion animal suspected to have an atopic dermatitis condition, and having means adapted to execute the steps of the said method; or alternatively both offline and online.

Hence, according to one embodiment, the disclosure relates to a device for determining a cutaneous lesion score of a companion animal suspected to have an atopic dermatitis condition, using at least:
- at least one lesions global image of said companion animal body surface, and
- optionally, at least one metadata relative to said companion animal, and
- optionally, at least one lesion specific image of a cutaneous lesion of said companion animal,

the device including an assessment module trained beforehand to learn features indicative of an atopic dermatitis condition in a companion animal, based at least on a plurality of previously acquired images of companion animal body surfaces, some of these images having cutaneous lesion(s),
said trained assessment module being configured to operate on said at least one lesions global image and, optionally, said at least one metadata and/or lesion specific image of said companion animal, and to generate at least one score indicative of the cutaneous state of said companion animal.

Said device may include an acquisition module for acquiring said at least one lesions global image, and optionally said at least one lesion specific image, of said companion animal body surface.

In a particular embodiment, lesion images of the patient may be downloaded from a smartphone, notably by a veterinarian practitioner.

According to another aspect, the disclosure relates to a device for training an assessment module to learn features indicative of an atopic dermatitis condition in a companion animal, using at least a plurality of previously acquired images of companion animal body surfaces, some of these images having cutaneous lesion(s), the method including:
- extracting at least one lesion feature from each previously acquired image,
- associating at least said image and said at least one lesion feature to an animal cutaneous state, and
- training the assessment module to learn said association.

According to another embodiment, the disclosure relates to a computer program product for determining a cutaneous lesion score of a companion animal suspected to have an atopic dermatitis condition, using at least:
- an assessment module trained beforehand to learn features indicative of an atopic dermatitis condition in a companion animal, based at least on a plurality of previously acquired images of companion animal body surfaces, some of these images having cutaneous lesion(s),
- at least one lesions global image of said companion animal body surface, and
- optionally, at least one metadata relative to said companion animal, and
- optionally, at least one lesion specific image of a cutaneous lesion of said companion animal,
the computer program product including a support and stored on this support instructions that can be read by a processor, these instructions being configured to:
a) operating said trained assessment module on said at least one lesions global image and, optionally, said at least one metadata and/or lesion specific image of said companion animal, and
b) based on said assessment module, generating at least one score indicative of the cutaneous state of said companion animal.

According to another embodiment, the disclosure relates to a computer program product for training an assessment module to learn features indicative of an atopic dermatitis condition in a companion animal, using at least a plurality of previously acquired images of companion animal body surfaces, some of these images having cutaneous lesion(s), the method including:
- extracting at least one lesion feature from each previously acquired image,
- associating at least said image and said at least one lesion feature to an animal cutaneous state, and
- training the assessment module to learn said association.

According to another embodiment, the disclosure relates to a computer-readable medium having stored thereon one or both of the computer program products described above. Such a computer-readable medium may include, or may consist of, a physical embodiment of a collection of data including one to a plurality of data sets that can be configured in one to a plurality of databases. It may thus include, or may consist of, medium wherein or whereon such data can be stored. Such a computer-readable medium may also include or consist of more than one medium; however in such a case the media may be functionally linked.

A computer-aided system of the disclosure typically includes one or more user interface(s) enabling entry of an input data. Such input data may, for instance, include lesion image(s) of said companion animal body surface and metadata relative to said companion animal.

Embodiments of the disclosure and all of the functional operations described in this specification can be implemented in digital electronic circuitry, or in computer software, firmware, or hardware, including the structures disclosed in this specification and their structural equivalents, or in combinations of one or more of them. Embodiments of the disclosure can be implemented as one or more computer program products, i.e., one or more modules of computer program instructions encoded on a computer-readable medium for execution by, or to control the operation of, data processing apparatus.

The computer-readable medium can be a machine-readable storage device, a machine-readable storage substrate, a memory device, a composition of matter effecting a machine-readable propagated signal, or a combination of one or more of them. The term "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them. A propagated signal is an artificially generated signal, e.g., a machine-generated electrical, optical, or electromagnetic signal, that is generated to encode information for transmission to suitable receiver apparatus.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network

A computer can be embedded in another device, e.g., a mobile telephone, a personal digital assistant (PDA), a mobile audio player, a Global Positioning System (GPS) receiver, to name just a few. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the disclosure can be implemented on a computer or a smartphone having a display device for displaying information to the user and a keyboard and a pointing or tactile device by which the user can provide input to the computer.

Embodiments of the disclosure can be implemented in a computing system that can include a back-end component, e.g., as a data server, or that can include a middleware component, e.g., an application server, or that can include a front-end component, e.g., a client computer having a graphical user interface or a Web browser through which a user can interact with an implementation of the disclosure, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet.

According to a another aspect, the disclosure relates to_a computer-implemented method for determining a cutaneous lesion score of a companion animal suspected to have an atopic dermatitis condition, the method comprising: receiving at least one lesions global image of said companion animal body surface, and optionally at least one metadata relative to said companion animal and/or at least one lesion specific image of a cutaneous lesion of said companion animal and determining based on these data a cutaneous lesion score of said companion animal. In a particular embodiment, this method further comprises a step of transmitting the cutaneous lesion score of the companion animal to a mobile device.

According to a seventh aspect, the disclosure relates to a computer-implemented method for determining a cutaneous lesion score of a companion animal suspected to have an atopic dermatitis condition, the method comprising: receiving at least one lesions global image of said companion animal body surface, and optionally at least one metadata relative to said companion animal and/or at least one lesion specific image of a cutaneous lesion of said companion animal, determining based on these data a cutaneous lesion score of said companion animal and determining a recommendation to a pet owner based on the score. In a particular embodiment, said recommendation is a nutritional recommendation. In another particular embodiment, this method further comprises a step of transmitting the recommendation to a mobile device of the pet owner.

Based on the score, a recommendation can be determined and transmitted to one or more of a pet owner, a veterinarian, a researcher and/or any combination thereof. The recommendation, for example, can include one or more health recommendations for preventing the companion animal from developing one or more of a disease, a condition, an illness and/or any combination thereof. The recommendation, for example, can include one or more of: a food product, a pet service, a supplement, an ointment, a drug to improve the wellness or health of the pet, a pet product, and/or any combination thereof. In other words, the recommendation can be a nutritional recommendation. In some embodiments, a nutritional recommendation can include an instruction to feed a companion animal one or more of: a chewable, a supplement, a food and/or any combination thereof. In some embodiments, the recommendation can be a medical recommendation. For example, a medical recommendation can include an instruction to apply an ointment to a companion animal, to administer one or more drugs to a companion animal and/or to provide one or more drugs for or to a companion animal. The term "pet product" can include, for example, without limitation, any type of product, service, or equipment that is designed, manufactured, and/or intended for use by a companion animal. For example, the pet product can be a toy, a chewable, a food, an item of clothing, a collar, a medication, a health tracking device, a location tracking device, and/or any combination thereof. In another example a pet product can include a genetic or DNA testing service for pets. The term "pet owner" can include any person, organization, and/or collection of persons that owns and/or is responsible for any aspect of the care of a companion animal.

### EXAMPLES

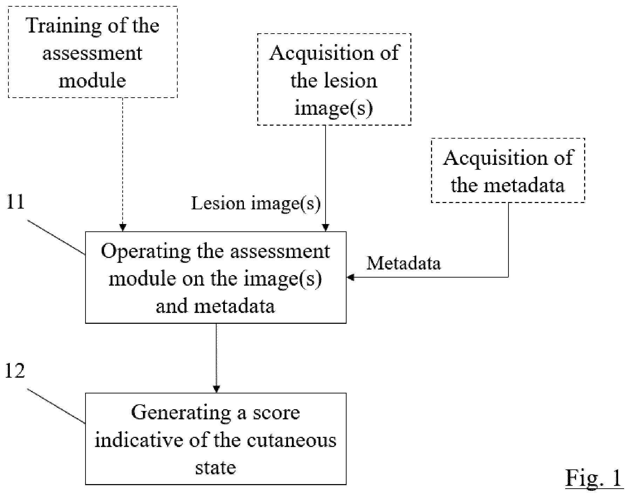

Various steps in an example of a method, according to the disclosure, for determining a cutaneous lesion score of a companion animal suspected to have an atopic dermatitis condition, are depicted in **figure 1** and will be described in detail in what follows. The method uses an assessment module trained beforehand to learn features indicative of an atopic dermatitis condition in a companion animal, based at least on a plurality of previously acquired images of companion animal body surfaces, some of these images having cutaneous lesion(s). The method also uses at least one lesions global image of said companion animal body surface, acquired beforehand, and at least one metadata relative to said companion animal, acquired beforehand as well.

During a step 11, said trained assessment module is operated on said at least one lesions global image and said at least one metadata of said companion animal. During a step 12, based on said assessment module, a score indicative of the cutaneous state of said companion animal is generated.

In the illustrated example, the method uses at least one lesions global image which is an image of the underside body surface of said companion animal including said at least one cutaneous lesion, and also one lesion specific image which is an enlarged image of at least one area of interest with said at least one cutaneous lesion.

Said at least one metadata relative to said companion animal may include animal data, such as breed, species, activity level, medical history, reproductive status, age, gender, weight, spayed or neutered status, a biological value from a biological sample, body condition, health status, lifestyle, habitat, coat information, or risk factor, and/or medical data, such as the age of disease onset, the existence of previous episodes of hotspots, urticaria or angioedema, the presence of cortico-response pruritus, the excess of hair loss, scaling or dryness, gastrointestinal signs, an indication on whether or not symptoms worsen after walking in grass, or medical history of chronic and/or recurrent dermatoses or otitis.

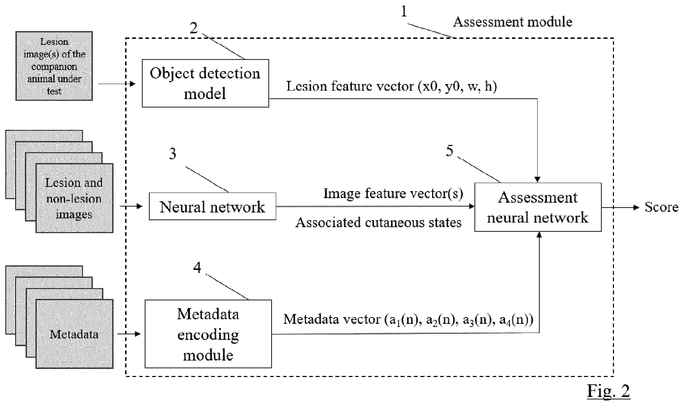

As illustrated in **figure 2****,** the assessment module 1, uses a predictive model which include an object detection model 2, a neural network 3, especially a convolutional neural network, a metadata encoding module 4 and an assessment neural network 5.

In the illustrated example, the object detection model 2 is based on YOLOv3 with the DarkNet architecture and performs a recognition analysis on said at least one lesion image to reference said at least one cutaneous lesion, several lesion features are referenced by an expert, the origin coordinates x0, y0, the height h, the width w, and a lesion feature vector is formed.

In the illustrated example, the neural network 3 is based on the opensource model InceptionV3 and is trained beforehand to recognize cutaneous states of companion animals based at least on one lesion feature extracted from said plurality of previously acquired images of companion animal body surfaces and associated with known animal cutaneous states. Image feature vectors are formed and are associated with said known animal cutaneous states.

For the training of the neural network 3 of the assessment module 1, at least 100 images are used, some of companion animals with cutaneous lesions, some of healthy animals and some animals with features other than atopic dermatosis. The selection includes different ages, behaviors, all different colors of hair, cross-modification with aging, all different hair density and composition (primary/intermediate/secondary hairs). The distribution of animals has to be as large as possible to have the most important database. For the images with lesions, the largest variety of locations possible is chosen.

In the illustrated example, the metadata encoding module 4 is configured to convert said metadata, that is to say the answers ai(n) to questions qi, into a categorical vector a₁(1)... ai(n), with i the number of the question and n the number of predefined answers.

In the illustrated example, the assessment neural network 5 is a single-layer perceptron artificial neural network and uses said lesion feature vector of object detection model 2, said image feature vector(s) of neural network 3 and said metadata vector of metadata encoding module 4 to generate the score indicative of the cutaneous state of said companion animal, based on the vectors learned beforehand and associated with known animal cutaneous states.

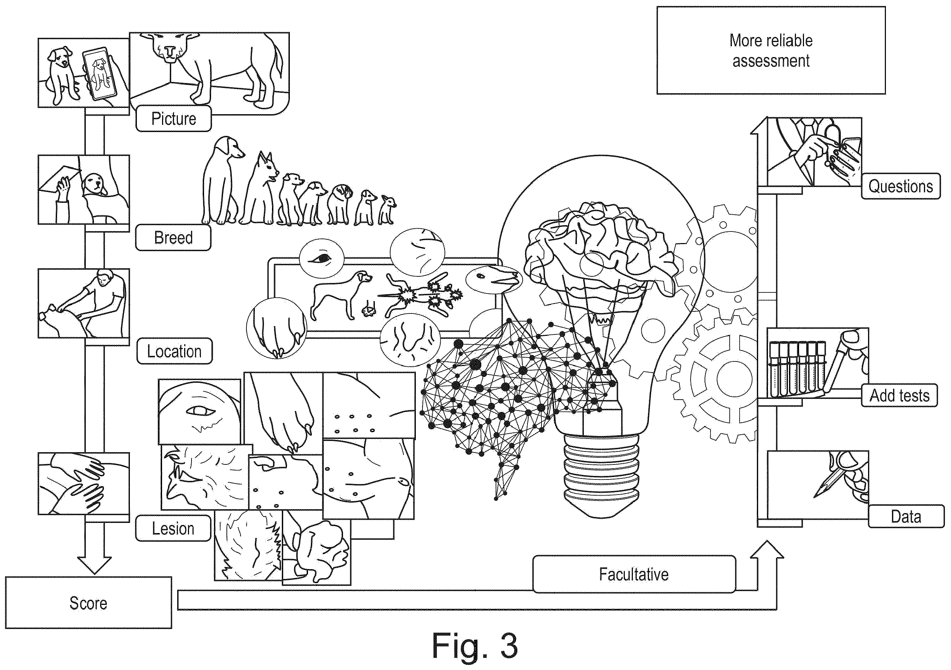

**Figure 3** illustrates an exemplary use of the method according to the disclosure. At least one image of the companion animal may be taken by the general veterinarian practitioner during a consultation. Metadata as the breed of the animal, features about the lesion, and its location are taken into account to obtain a score indicative of the cutaneous state of said companion animal, as described above.

Some facultative steps can lead to a more reliable assessment, as the use of more data, more tests or questions.

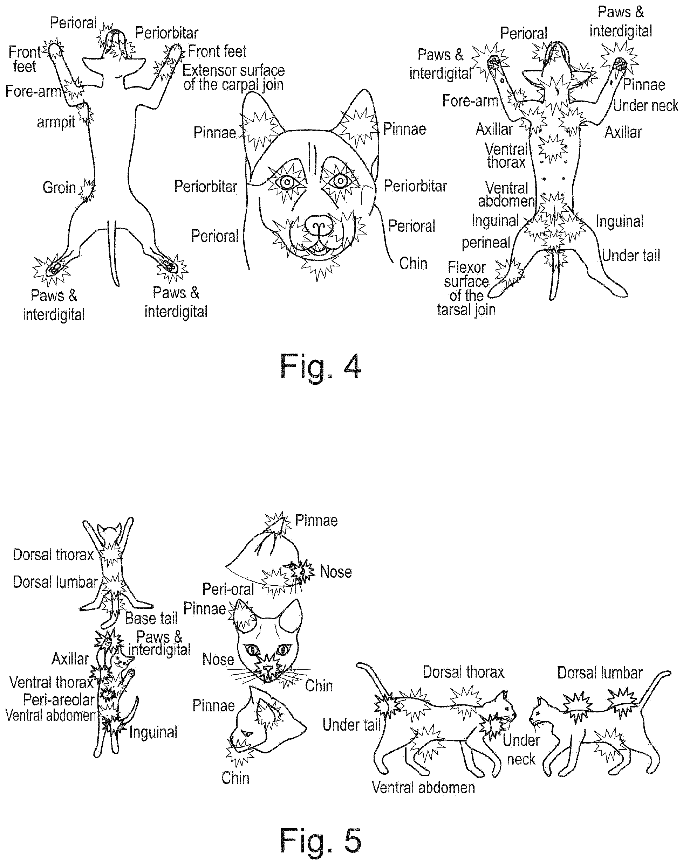

**Figure 4** shows some of the most commonly areas in a dog affected with an atopic dermatitis condition.

**Figure 5** shows some of the most commonly areas in a cat affected with an atopic dermatitis condition.

The most commonly affected areas are thus the head, especially the ear, perioral and/or periocular areas, the legs, especially the front feet and/or the interdigital areas, the flexor surface of the tarsal joint and/or the extensor surface of the carpal joint, and the trunc, especially the groin, armpit, ventral and/or perineal areas.

**Figure 6** shows an example of use of the method according to the invention through a smartphone application. Figure 6(a) shows a user interface enabling the animal owner to entry some input data, namely metadata relative to said companion animal such as its known breed, how long ago the alleged dermatitis onset occurred, indoor or outdoor life, chronic or recurrent dermatitis or behaving as a permanent background, known atopic dermatitis condition or not. Then, a lesions global image of the body surface of the animal is downloaded in the interface. The areas showing lesions may be marked out, as shown in figure 6(b).

The pet owner, as shown in figure 6(c), is asked to choose the area(s) of its body surface showing the most characteristic lesion(s), for instance head, armpit, groin or foot. Then, a lesion specific image corresponding to an enlarged lesion image(s) of said companion animal body surface are thus downloaded in the interface, as shown in figure 6(d). As explained above, all of these input data allow generating a score indicative of the cutaneous state of the animal, which may be shown on a subsequent interface.

It should be appreciated that in the above description of exemplary embodiments of the disclosure, various features of the disclosure are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the disclosure requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this disclosure.

Furthermore, while some embodiments described herein include some but not other features included in other embodiments, combinations of features of different embodiments are meant to be within the scope of the disclosure, and form different embodiments, as would be understood by those skilled in the art. For example, in the following claims, any of the claimed embodiments can be used in any combination.

Thus, while certain embodiments have been described, those skilled in the art will recognize that other and further modifications may be made thereto without departing from the spirit of the disclosure, and it is intended to claim all such changes and modifications as falling within the scope of the disclosure. For example, functionality may be added or deleted from the block diagrams and operations may be interchanged among functional blocks. Steps may be added or deleted to methods described within the scope of the present disclosure

The above disclosed subject matter is to be considered illustrative, and not restrictive, and the appended claims are intended to cover all such modifications, enhancements, and other implementations, which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description. While various implementations of the disclosure have been described, it will be apparent to those of ordinary skill in the art that many more implementations are possible within the scope of the disclosure. Accordingly, the disclosure is not to be restricted except in light of the attached claims and their equivalents

### [REFERENCES]

Genovese D.W. & al. Histological and dermatoscopic description of sphynx cat skin. Vet Dermatol 2014;25:523-e90.
Scarampella F. & al. Dermoscopic features in 35 dogs with juvenile-onset demodecosis and 35 breed- and age-matched dogs: an observational descriptive study. Vet Dermatol 2014;25:383-405.
Zanna G. & al. Dermoscopic evaluation of skin in healthy cats. Vet Dermatol 2015;26:14-e4.
Scarampella F. & al. Dermoscopic features in 12 cats with dermatophytosis and 12 cats with self-induced alopecia due to other causes: an observational descriptive study. Vet Dermatol 2015;26:282-e63.
Zanna G. & al. The usefulness of dermoscopy in canine pattern alopecia: a descriptive study. Vet Dermatol 2017;28:161-e34.
Guaguere E. & al. A practical guide to Canine Dermatology. Merial 2008; 579pp.

## Claims

1. A method for determining a cutaneous lesion score of a companion animal suspected to have an atopic dermatitis condition, using an assessment module trained beforehand to learn features indicative of an atopic dermatitis condition in a companion animal, based at least on a plurality of previously acquired images of companion animal body surfaces, some of these images having cutaneous lesion(s),
the method comprising at least the steps of:
a) receiving companion animal data corresponding to said companion animal, wherein the companion animal data include at least one global lesions image of said companion animal, and at least one metadata relative to said companion animal,
b) operating said trained assessment module on the companion animal data, and
c) based on said assessment module, generating a cutaneous lesion score indicative of at least one cutaneous state of said companion animal.

2. The method of claim 1, wherein said at least one global lesions image comprise at least one image of the underside body surface of said companion animal.

3. The method of claim 1 or 2, wherein said companion animal data further comprise at least one lesion specific image of a cutaneous lesion of said companion animal.

4. The method of claim 3, wherein said at least one lesion specific image comprises at least an enlarged image of at least one area of interest including said at least one cutaneous lesion.

5. The method of any one of the preceding claims, wherein said at least one metadata relative to said companion animal includes animal data, such as breed, species, activity level, medical history, reproductive status, age, gender, weight, spayed or neutered status, a biological value from a biological sample, body condition, health status, lifestyle, habitat, coat information, or risk factor, and/or medical data, such as the age of disease onset, the existence of previous episodes of hotspots, urticaria or angioedema, the presence of cortico-response pruritus, the excess of hair loss, scaling or dryness, gastrointestinal signs, an indication on whether or not symptoms worsen after walking in grass, or medical history of chronic and/or recurrent dermatoses or otitis.

6. The method of any one of the preceding claims, using a plurality of metadata relative to said companion animal, and especially comprising the step of providing a pathological profile of said companion animal based on said plurality of metadata.

7. The method of any one of the preceding claims, wherein said assessment module uses a predictive model, comprising an object detection model, a neural network, especially a convolutional neural network, and a metadata encoding module.

8. The method of the preceding claim, wherein said object detection model performs a recognition analysis on said at least one lesion image to reference said at least one cutaneous lesion, one or more lesion features being especially referenced, such as spatial features, as the origin coordinates, the height, and/or the width.

9. The method of any one of claims 6 or 7, wherein said neural network is trained beforehand to recognize cutaneous states of companion animals based at least on one lesion feature extracted from said plurality of previously acquired images of companion animal body surfaces.

10. The method of the preceding claim, wherein said generated score depends at least on an assessed correlation between said lesion features and said lesion image.

11. The method of any one of the preceding claims, wherein an additional lesion image is used, especially an optical zoomed image of said lesion.

12. The method of any one of the preceding claims, further comprising the step of providing to a user interface said score relative to said companion animal and generated at step b).

13. The method of any one of the preceding claims, wherein said areas of said companion animal body surface are chosen among the head, especially the ear, perioral and/or periocular areas, the legs, especially the front feet and/or the interdigital areas, the flexor surface of the tarsal joint and/or the extensor surface of the carpal joint, and the trunc, especially the groin, armpit, ventral and/or perineal areas.

14. A method for training an assessment module to learn features indicative of an atopic dermatitis condition in a companion animal, using at least a plurality of previously acquired images of companion animal body surfaces, some of these images having cutaneous lesion(s), the method comprising:
- extracting at least one lesion feature from each previously acquired image,
- associating at least said image and said at least one lesion feature to an animal cutaneous state, and
- training the assessment module to learn said association.

15. The method of the preceding claim, wherein, said assessment module comprising at least one neural network, the method comprising the step of updating weights of the neural network according to said association between said at least one lesion feature and said animal cutaneous state.

16. A device for determining a cutaneous lesion score of a companion animal suspected to have an atopic dermatitis condition,
the device comprising an assessment module trained beforehand to learn features indicative of an atopic dermatitis condition in a companion animal, based at least on a plurality of previously acquired images of companion animal body surfaces, some of these images having cutaneous lesion(s),
said trained assessment module being configured to operate on the companion animal data wherein the companion animal data includes at least one lesions global image of said companion animal body surface, and at least one metadata relative to said companion animal, , and to generate a cutaneous lesion score indicative of at least one cutaneous state of said companion animal.

17. The device of the preceding claim, comprising an acquisition module for acquiring said at least one lesion image of said companion animal body surface.

18. Computer program product for determining a cutaneous lesion score of a companion animal suspected to have an atopic dermatitis condition, using an assessment module trained beforehand to learn features indicative of an atopic dermatitis condition in a companion animal, based at least on a plurality of previously acquired images of companion animal body surfaces, some of these images having cutaneous lesion(s), the computer program product comprising a support and stored on this support instructions that can be read by a processor, these instructions being configured to:
a) receiving companion animal data corresponding to said companion animal, wherein the companion animal data includes at least one lesions global image of said companion animal body surface, and at least one metadata relative to said companion animal,
b) operating said trained assessment module on the companion animal data, and
c) based on said assessment module, generating a cutaneous lesion score indicative of at least one cutaneous state of said companion animal.
